# EUROPEAN PATENT APPLICATION

(11) **EP 3 607 889 A1**
(43) Date of publication of application: **12.02.2020**
(21) Application number: 19190126.3
(22) Date of filing: 05.08.2019
(51) Int. Cl.: A61B 17/02, A61F 5/445

(54) **STOMAL SUPPORT DEVICE**

(30) Priority: 06.08.2018 US 201862715022 P; 22.05.2019 US 201916419046
(71) Applicant: Covidien LP, Mansfield, MA 02048 (US)
(72) Inventor: AVVLN, Srinivasa Murthy Aravalli, 500049 Hyderabad (IN)
(74) Representative: Soames, Candida Jane

(57) **Abstract**

A stomal device includes a rod and first and second anchoring portions configured to detachably support the rod. The rod is configured to be positioned in a loop of a body vessel to support at least a portion of the body vessel on an abdominal wall. The rod includes first and second end portions having respective first and second connecting portions. The first and second anchoring portions include third and fourth connecting portions configured to detachably mate with the first and second connecting portions of the first and second end portions of the rod, respectively, the first and second anchoring portions transversely extending from the rod.

## Description

### CROSS-REFERENCE TO RELATED APPLICATION

This application claims the benefit of and priority to U.S. Provisional Patent Application No. 62/715,022 filed August 6, 2018, the entire disclosure of which is incorporated by reference herein.

### BACKGROUND

### 1. Technical Description

The present disclosure is directed to a stomal support device and to method for performing an ostomy procedure and, more particularly, to a modular stomal support device and a method of use thereof.

### 2. Background of Related Art

During an ostomy procedure, a portion of an internal body vessel, such as the intestine or colon, is exteriorized to form a stoma. Stomas may be created in conjunction with an ostomy procedure by securing a bisected portion of the internal body vessel to the abdominal wall to provide internal access into the internal body vessel for collecting fecal matter. Ostomy surgery is sometimes performed on an emergency basis due to diverticulitis, trauma, radiation complications, volvulus, necrotic bowel, bowel perforation, etc. An ostomy may be temporary to allow for healing of the bowel or a decrease of inflammation at a surgical site. In some instances, an ostomy may be permanent.

In ostomy procedures, the internal body vessel is secured to the abdominal wall and/or cutaneous tissue of the abdomen. Securing the internal body vessel to the abdominal wall and/or the cutaneous tissue of the abdomen keeps the stoma in the desired location and prevents it from withdrawing back into the abdominal cavity. Typically, a colostomy bag is connected to the stoma site with an adhesive to collect waste materials from the internal body vessel. Although a stoma has no sensory nerve endings and is insensitive to pain, several complications can result at the stoma site where the colostomy bag is secured, such as leaks, skin irritation, infection, etc. As such, the condition of the stoma must be assessed regularly.

Thus, there is a continuing need in the medical arts for an alternative mechanism for collecting fecal matter or waste material from an internal body vessel that overcomes the above disadvantages and can improve the quality of life of patients requiring ostomy.

### SUMMARY

In accordance with an embodiment of the present disclosure, a stomal device includes a rod and first and second anchoring portions configured to detachably support the rod. The rod is configured to be positioned in a loop of a body vessel to support at least a portion of the body vessel on an abdominal wall. The rod includes first and second end portions having respective first and second connecting portions. The first and second anchoring portions include third and fourth connecting portions configured to detachably mate with the first and second connecting portions of the first and second end portions of the rod, respectively. The first and second anchoring portions transversely extend from the rod.

In an embodiment, the first and second anchoring portions may define respective longitudinal axes.

In another embodiment, at least one of the first or second anchoring portions may include an arcuate profile.

In yet another embodiment, the arcuate profile of the at least one of the first or second anchoring portions may have a concavity.

In still yet another embodiment, the arcuate profile of the at least one of the first or second anchoring portions may have a convexity.

In still yet another embodiment, at least one of the first or second anchoring portions may include a padding portion.

In yet another embodiment, the padding portion may be formed of a foam.

In yet another embodiment, the padding portion may include an adhesive layer.

In still yet another embodiment, the first and second connecting portions of the rod and the third and fourth connecting portions of the first and second anchoring portions may include dove-tail configuration.

In accordance with another embodiment of the present disclosure, a surgical kit includes a rod, a pair of first anchoring portions configured to detachably support the rod, a pair of second anchoring portions configured to detachably support the rod, and a pair of third anchoring portions configured to detachably support the rod. The rod is configured to be positioned in a loop of a body vessel to support at least the loop of the body vessel on an abdominal wall. The rod includes first and second end portions. The first anchoring portions are transversely connectable with the respective first and second end portions of the rod. The first anchoring portions each define a longitudinal axis. The second anchoring portions are detachably connectable with the respective first and second end portions of the rod. The second anchoring portions include an arcuate profile having concavity. The third anchoring portions extend transversely from the respective first and second ends of the rod. The third anchoring portions include an arcuate profile having convexity.

In an embodiment, the first anchoring portions may include connecting portions configured to detachably mate with the respective first and second end portions of the rod.

In another embodiment, the second or third anchoring portions may include connecting portions configured to detachably mate with the respective first and second end portions of the rod.

In yet another embodiment, each of the first anchoring portions may include padding portions at opposing ends thereof.

In still yet another embodiment, the padding portions may be formed of a foam.

In still yet another embodiment, each of the padding portions may include an adhesive layer.

In accordance with another aspect of the present disclosure, a surgical method includes making an abdominal incision into an abdomen of a patient to provide access to an abdominal cavity of the patient; extracting a loop of a body vessel from the abdominal cavity through the abdominal incision; positioning a rod across the abdominal incision between the loop of the body vessel and the abdomen of the patient to inhibit the loop of the body vessel from receding back into the abdominal cavity of the patient through the abdominal incision; detachably supporting the rod by connecting first and second anchoring portions to respective ends of the rod; making a first incision into the body vessel to define a first opening within the body vessel; making a second incision into the body vessel to define a second opening within the body vessel; and replacing the first and second anchoring portions with third and fourth anchoring portions having a profile different than a profile of the first and second anchoring portions.

In an embodiment, replacing the first and second anchoring portions may include providing the third and fourth anchoring portions each having a concavity.

In another embodiment, replacing the first and second anchoring portions may include providing the third and fourth anchoring portions each having a convexity.

In yet another embodiment, the method may further include securing the first and second anchoring portions to the abdomen of the patient by an adhesive on padding portions on the first and second anchoring portions.

In still yet another embodiment, the method may further include suturing a portion of the body vessel to the abdomen of the patient.

### BRIEF DESCRIPTION OF THE DRAWINGS

Various embodiments of the presently disclosed stomal device are described herein below with reference to the drawings, wherein:
FIG. 1 is a top perspective view of a stomal device in accordance with an embodiment of the present disclosure;
FIG. 2 is a bottom perspective view of the stomal device of FIG. 1;
FIG. 3 is an exploded perspective view of the stomal device of FIG. 1 with parts separated;
FIG. 4 is a top perspective view of a stomal device in accordance with another embodiment of the present disclosure;
FIG. 5 is a bottom perspective view of the stomal device of FIG. 4;
FIG. 6 a top perspective view of a stomal device in accordance with another embodiment of the present disclosure;
FIG. 7 is a bottom perspective view of the stomal device of FIG. 6;
FIG. 8 is a top perspective view of a stomal device in accordance with another embodiment of the present disclosure;
FIG. 9 is a kit including the stomal devices of FIGS. 1-8;
FIG. 10 is a perspective view of a surgical site of a patient; and
FIGS. 11 and 12 are top views of the stomal devices of FIGS. 1-8 illustrating use thereof.

### DETAILED DESCRIPTION OF EMBODIMENTS

The presently disclosed stomal device will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

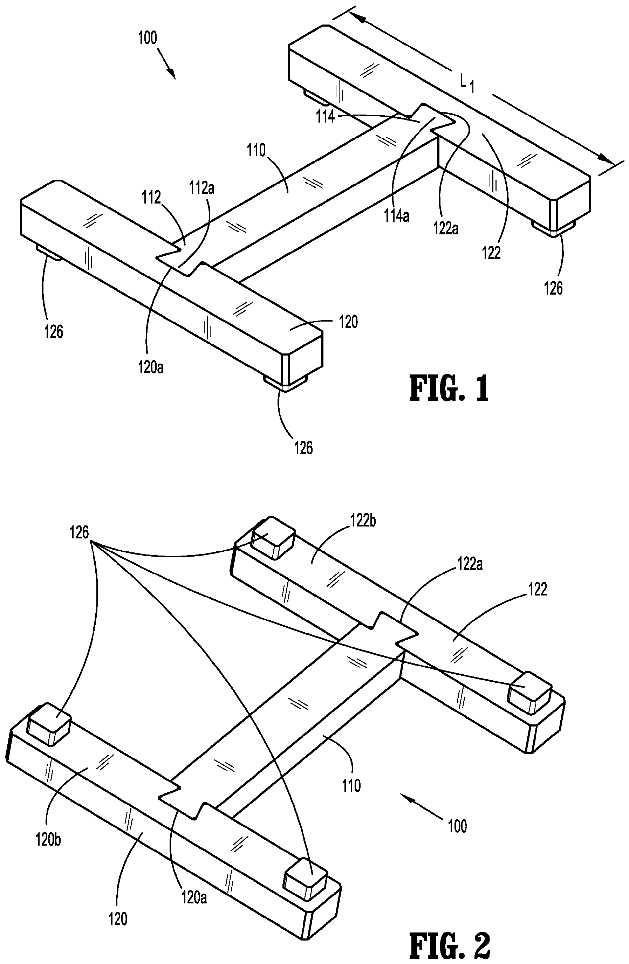

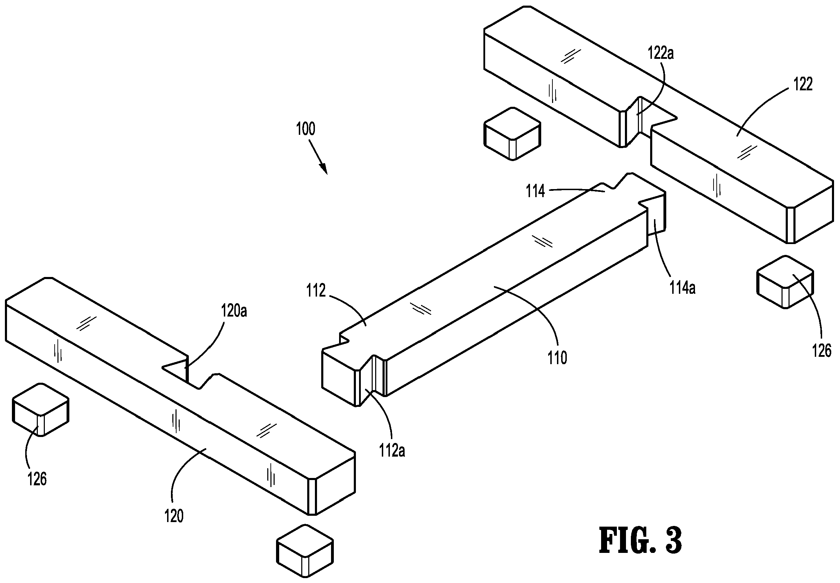

With reference to FIGS. 1 and 2, the presently disclosed stomal device is shown generally as a stomal device 100 and is configured for use in, e.g., loop colostomy, in which, a part of the colon is lifted above skin level and held in place with the stomal device 100. However, it is also contemplated that the stomal device 100 may be used in other surgical procedures such as, e.g., ileostomy or urostomy. The stomal device 100 includes a rod 110 and first and second anchoring portions 120, 122 detachably coupled to the rod 110. The first and second anchoring portions 120, 122 may be transversely connected to respective opposing first and second ends 112, 114 of the rod 110. For example, the first and second ends 112, 114 of the rod 110 may have respective dove-tail connections 112a, 114a that frictionally mate with respective dove-tail connections 120a, 122a (FIG. 3) of the first and second anchoring portions 120, 122. Under such a configuration, the first and second anchoring portions 120, 122 are frictionally secured with the rod 110 to provide a detachable connection with the rod 110.

With reference to FIG. 3, the first and second anchoring portions 120, 122 may include padding portions 126 formed of, e.g., foam, configured to engage the abdominal wall or the skin of the patient. The padding portions 126 may reduce concentration of weight or stress on the abdominal wall or the skin when the stomal device 100 is supported on the abdominal wall or the skin. It is contemplated that the padding portions 126 may be disposed over the entire contacting surfaces 120b, 122b (FIG. 2) of the respective first and second anchoring portions 120, 122 or, as shown, just on selected portions of the contacting surfaces 120b, 122b.

In embodiments, the rod 110 may have any suitable cross-sectional shape such as, e.g., a semi-circular, circular, square, rectangular, or the like. In some embodiments, the rod 110 may be formed from pliable, semi-rigid, or rigid material, such as a plastic, a polymer, a metal, or the like. In certain embodiments, each padding portion 126 may include an adhesive (not shown) in the form of, e.g., a flat sheet, sticker, tape, or the like, to facilitate securement of the stomal device 100 on abdominal wall or the skin of the patient. In embodiments, the adhesive may include any type of biocompatible adhesive (e.g., glue). In some embodiments, the contacting surfaces 120b, 122b (FIG. 2) of the first and second anchoring portions 120, 122 may optionally include an adhesive coating in lieu of the adhesive on the padding portion 126.

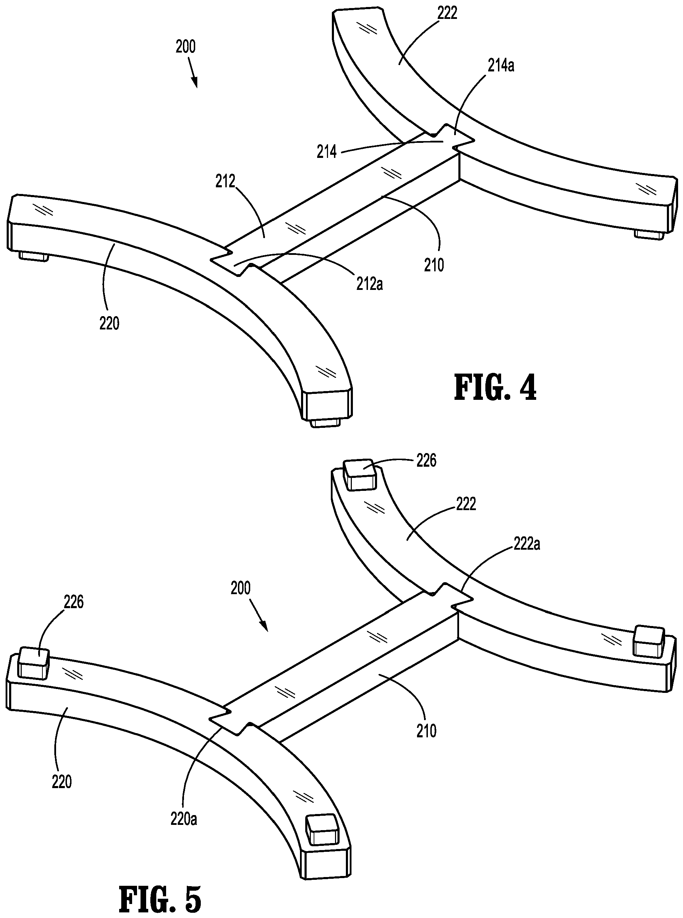

With reference now to FIGS. 4 and 5, a stomal device in accordance with another embodiment of the present disclosure is shown generally as a stomal device 200. The stomal device 200 is substantially identical to the stomal device 100, and thus, identical parts in the stomal device 200 will not be described herein to avoid obscuring the present disclosure in unnecessary detail. The stomal device 200 includes a rod 210 and first and second anchoring portions 220, 222 detachably coupled to the rod 210. The rod 210 may be identical to the rod 110 (FIG. 1), and thus, may be interchangeably used. The first and second anchoring portions 220, 222 each have an arcuate profile defining a concavity. The rod 210 includes opposing first and second ends 212, 214 configured to detachably engage the respective first and second anchoring portions 220, 222. For example, the first and second ends 212, 214 of the rod 210 have respective dove-tail connections 212a, 214a that frictionally mate with respective dove-tail connections 220a, 222a of the first and second anchoring portions 220, 222. Under such a configuration, the first and second anchoring portions 220, 222 are frictionally secured with the rod 110.

The rod 210 of the stomal device 200 may be identical to the rod 110 of the stomal device 100. Under such a configuration, the first and second anchoring portions 220, 222 may be used interchangeably with the first and second anchoring portions 120, 122. Due to the concavity of the first and second anchoring portions 220, 222, the first and second anchoring portions 220, 222 contact different portions of the abdominal wall or the skin of the patient when connected to the rod 110 or the rod 210, compared to the first and second anchoring portions 120, 122. Thus, during prolonged use of the stomal device 100, trauma or stress to the abdominal wall or the skin may be reduced when the first and second anchoring portions 120, 122 are replaced with the first and second anchoring portions 220, 222.

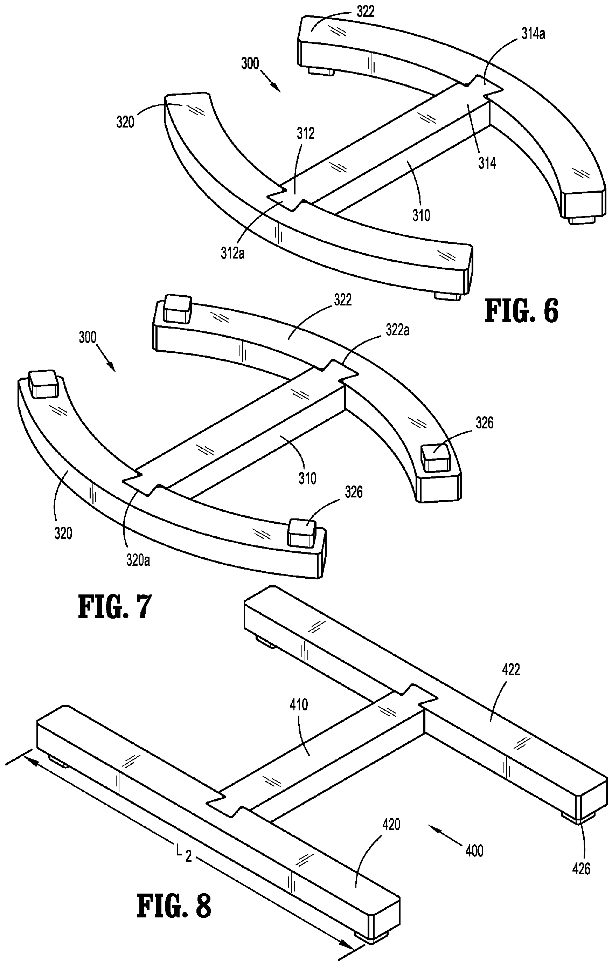

With reference now to FIGS. 6 and 7, a stomal device in accordance with another embodiment of the present disclosure is shown generally as a stomal device 300. The stomal device 300 is substantially identical to the stomal device 200, and thus, identical parts in the stomal device 300 will not be described herein to avoid obscuring the present disclosure in unnecessary detail. The stomal device 300 includes a rod 310 and first and second anchoring portions 320, 322 detachably coupled to the rod 310. The first and second anchoring portions 320, 322 each have an arcuate profile defining a convexity. The rod 310 includes opposing first and second ends 312, 314 configured to detachably engage respective first and second anchoring portions 320, 322. For example, the first and second ends 312, 314 of the rod 310 may have respective dove-tail connections 312a, 314a that frictionally mate with respective dove-tail connections 320a, 322a of the first and second anchoring portions 320, 322. Under such a configuration, the first and second anchoring portions 320, 322 are frictionally secured with the rod 310. The rod 310 of the stomal device 300 may be identical to the rod 110 of the stomal device 100. Under such a configuration, the first and second anchoring portions 320, 322 may be used interchangeably with the first and second anchoring portions 120, 122 of the stomal device 100. Due to the convexity of the first and second anchoring portions 320, 322, the first and second anchoring portions 320, 322 contact different portions of the abdominal wall or the skin of the patient when connected to the rod 110 or the rod 310, compared to the first and second anchoring portions 120, 220 and 122, 222. Thus, during prolonged use of the stomal device 100, trauma or stress to the abdominal wall or the skin may be reduced by replacing the first and second anchoring portions 120, 220 and 122, 222 with the first and second anchoring portions 320, 322.

With reference now to FIG. 8, a stomal device in accordance with another embodiment of the present disclosure is shown generally as a stomal device 400. The stomal device 400 is substantially identical to the stomal device 100, and thus, identical parts in the stomal device 400 will not be described herein to avoid obscuring the present disclosure in unnecessary detail. The stomal device 400 includes a rod 410 and first and second anchoring portions 420, 422 detachably coupled to the rod 410. In particular, the first and second anchoring portions 420, 422 each have a length L₂ larger than a length L₁ of the first and second anchoring portions 120, 122 of the stomal device 100. The rod 410 of the stomal device 400 may be identical to the rod 110 of the stomal device 100. Under such a configuration, the first and second anchoring portions 420, 422 may be used interchangeably with the first and second anchoring portions 120, 122 of the stomal device 100. Due to the difference in length L₂ of the first and second anchoring portions 420, 422 and the length L₁ (FIG. 1) of the first and second anchoring portions 120, 122, the first and second anchoring portions 420, 422 contact different portions of the abdominal wall or the skin of the patient when attached to the rod 110 or the rod 410, compared to the first and second anchoring portions 120, 220, 320 and 122, 222, 322. Thus, trauma or stress to the abdominal wall or the skin during prolonged use of the stomal device 100 may be reduced by periodically replacing the first and second anchoring portions 120, 122 with the first and second anchoring portions 420, 422.

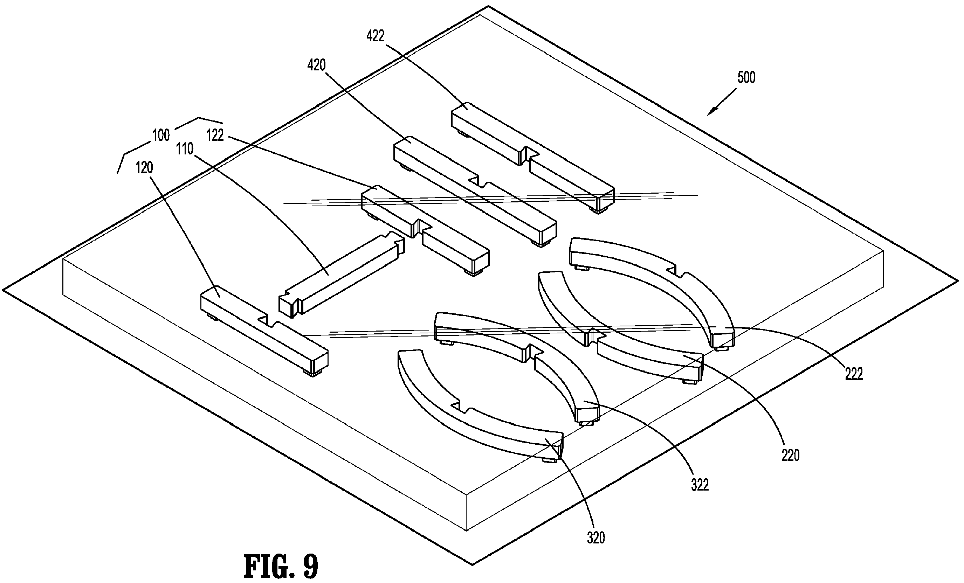

With reference now to FIG. 9, the stomal device 100 may be provided in a kit 500 to enable the clinician to interchangeably utilize different first and second anchoring portions 120, 220, 320, 420 and 122, 222, 322, 422. Various first and second anchor portions 120, 220, 320, 420 and 122, 222, 322, 422 may support the rod 110 on different parts of the abdominal wall or the skin of the patient, thereby reducing stress and trauma to the abdominal wall or the skin in prolonged use of the rod 110. In addition, the stomal devices 100 may also reduce infection by enabling the clinician to replace the first and second anchor portions 120, 220, 320, 420 and 122, 222, 322, 422 during prolonged use of the stomal devices 100, 200, 300, 400. In this manner, sores and wounds on the skin at the support site may be reduced.

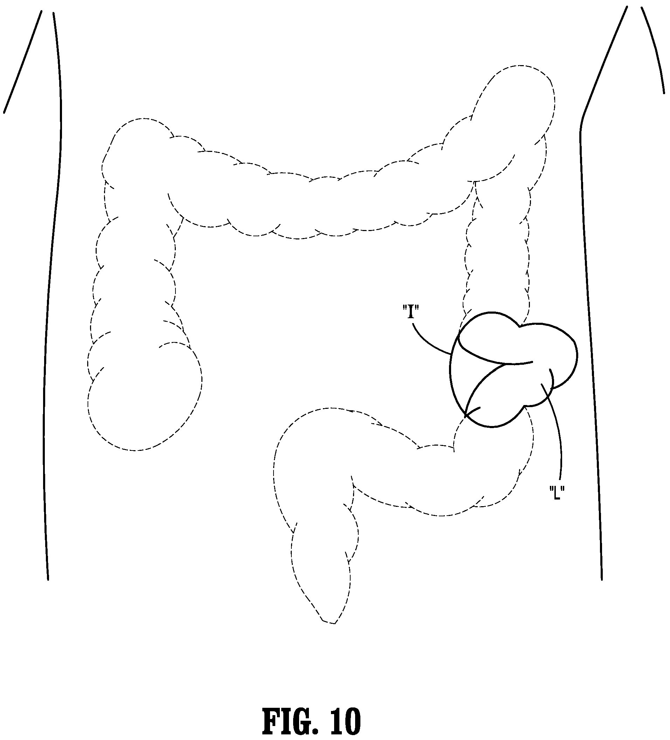

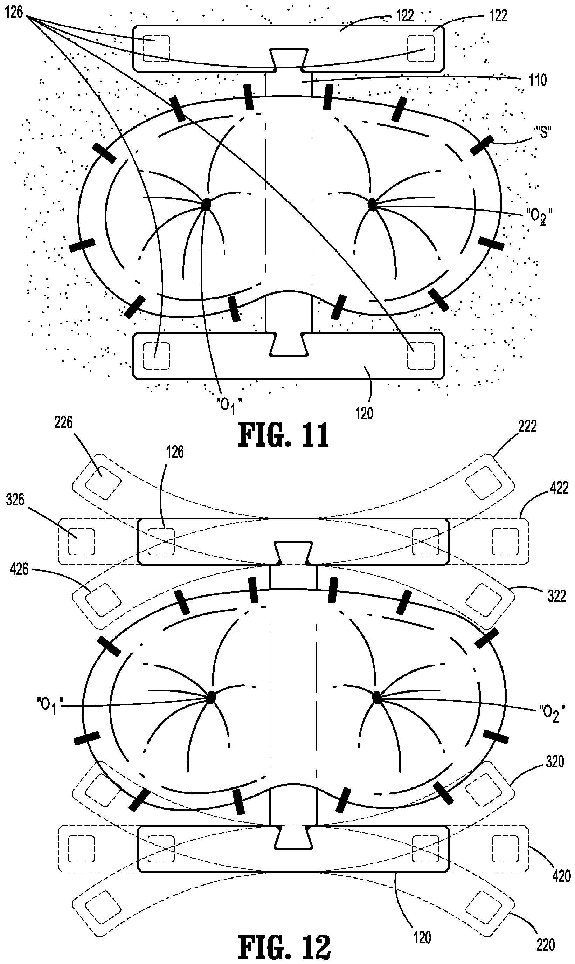

With reference to FIGS. 10-12, in use, for example, in a loop ostomy procedure (e.g., a diverting sigmoid loop colostomy, loop ileostomy, etc.), an incision is made into a patient's abdomen at an incision site "I." A portion or loop "L" of the body vessel is pulled outwardly through the incision site "I" such that the loop "L" extends out of the patient's abdomen. The body vessel may include the bowel, e.g., small or large intestine, colon, or rectum. In order to inhibit the loop "L" from receding through the incision site "I" and back into the abdominal cavity, the rod 110 is positioned centrally between the loop "L" and the patient's abdomen (e.g., at skin level).

The tissue contacting surfaces of the padding portion 126 of the first and second anchoring portions 120, 122 are brought into contact with the patient's abdomen adjacent the incision site "I" to secure the rod 110 to the patient's abdomen. With the rod 110 positioned between the loop "L" and the first and second ends 112, 114 (FIG. 1) of the rod 110 supported on the abdominal wall, the loop "L" of the body vessel is inhibited from receding back into the abdominal cavity. Thereafter, a first incision is made into the body vessel to create a first opening O₁ therein. For example, the first opening O₁ may be formed in the upstream portion of the large intestine. A second incision is made into the body vessel to create a second opening O₂ therein. The second opening O₂ may be formed within the body vessel, such as, e.g., the downstream portion of the intestine. At this time, the first and second anchoring portions 120, 122 are detachably connected to the rod 110 in order to support the rod 110 on the skin. The first and second openings O₁, O₂ define the stoma "ST." Each of the first and second openings O₁, O₂ is then affixed or sutured to the abdomen (e.g., at skin level) using one or more sutures "S" or adhesive (not shown). At this time, the clinician may attach a catheter (not shown) and/or a colostomy bag (not shown) to the stoma "ST", as needed. For example, as the patient undergoes normal digestive processes (e.g., excretion), digestive waste flows from the first opening O₁ of the stoma (e.g., the upstream portion of the intestine) through a first tube (not shown) and into the colostomy bag. A second tube (not shown) may separate the second opening O₂ of the stoma "ST" from the first opening O₁ of the stoma "ST," e.g., to keep the downstream portion of the intestine clean, and/or to allow for healing of the intestine. As such, no fluid can flow from the second tube of the catheter into the downstream portion of the intestine.

In order to inhibit possible skin irritation at the stoma "ST" after a prolonged use of the stomal rod 110 and the first and second anchoring portions 120, 122, the first and second anchoring portions 120, 122 may be replaced with other first and second anchoring portions 220, 320, 420 and 222, 322, 422 such that different parts of the abdominal wall or the skin engage the first and second anchoring portions 220, 320, 420 and 222, 322, 422 and support the rod 110. As discussed hereinabove, the first and second anchoring portions 120, 220, 320, 420 and 122, 222, 322, 422 may include padding materials 126, 226, 326, 426 which may include adhesive to further facilitate securement of the rod 110 to the skin. For example, by placing the padding materials 126, 226, 326, 426 on various locations on the abdominal wall or the skin of the patient relative to the original site of attachment, formation of sores or wounds may be reduced.

Persons skilled in the art will understand that the structures and methods specifically described herein and shown in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope or spirit of the disclosure. For example, while dove-tail configuration is used to detachably connect the first and second anchoring portions 120, 220, 320, 420 and 122, 222, 322, 422 to the rods 110, 210, 310, 410, it is also contemplated that other structures such as, e.g., snap-fit configuration, may be used.

Additionally, the elements and features shown or described in connection with certain embodiments may be combined with the elements and features of certain other embodiments without departing from the scope of the present disclosure, and that such modifications and variations are also included within the scope of the present disclosure. Accordingly, the subject matter of the present disclosure is not limited by what has been particularly shown and described.

The invention may be described by reference to the following numbered paragraphs:-
1. A stomal device comprising:
   a rod configured to be positioned in a loop of a body vessel to support at least a portion of the body vessel on an abdominal wall, the rod including first and second end portions having respective first and second connecting portions; and
   first and second anchoring portions configured to detachably support the rod, the first and second anchoring portions including third and fourth connecting portions configured to detachably mate with the first and second connecting portions of the first and second end portions of the rod, respectively, the first and second anchoring portions transversely extending from the rod.
2. The stomal device according to paragraph 1, wherein the first and second anchoring portions define respective longitudinal axes.
3. The stomal device according to paragraph 1, wherein at least one of the first or second anchoring portions includes an arcuate profile.
4. The stomal device according to paragraph 3, wherein the arcuate profile of the at least one of the first or second anchoring portions has a concavity.
5. The stomal device according to paragraph 3, wherein the arcuate profile of the at least one of the first or second anchoring portions has a convexity.
6. The stomal device according to paragraph 1, wherein at least one of the first or second anchoring portions includes a padding portion.
7. The stomal device according to paragraph 6, wherein the padding portion is formed of a foam.
8. The stomal device according to paragraph 6, wherein the padding portion includes an adhesive layer.
9. The stomal device according to paragraph 1, wherein the first and second connecting portions of the rod and the third and fourth connecting portions of the first and second anchoring portions include dove-tail configuration.
10. A surgical kit comprising:
   a rod configured to be positioned in a loop of a body vessel to support at least the loop of the body vessel on an abdominal wall, the rod including first and second end portions;
   a pair of first anchoring portions configured to detachably support the rod, the first anchoring portions transversely connectable with the respective first and second end portions of the rod, the first anchoring portions each defining a longitudinal axis;
   a pair of second anchoring portions configured to detachably support the rod, the second anchoring portions detachably connectable with the respective first and second end portions of the rod, the second anchoring portions including an arcuate profile having concavity; and
   a pair of third anchoring portions configured to detachably support the rod, the third anchoring portions extending transversely from the respective first and second ends of the rod, the third anchoring portions including an arcuate profile having convexity.
11. The surgical kit according to paragraph 10, wherein the first anchoring portions include connecting portions configured to detachably mate with the respective first and second end portions of the rod.
12. The surgical kit according to paragraph 10, wherein the second or third anchoring portions include connecting portions configured to detachably mate with the respective first and second end portions of the rod.
13. The surgical kit according to paragraph 10, wherein each of the first anchoring portions includes padding portions at opposing ends thereof.
14. The surgical kit according to paragraph 13, wherein the padding portions are formed of a foam.
15. The surgical kit according to paragraph 13, wherein each of the padding portions includes an adhesive layer.
16. A surgical method comprising:
   making an abdominal incision into an abdomen of a patient to provide access to an abdominal cavity of the patient;
   extracting a loop of a body vessel from the abdominal cavity through the abdominal incision;
   positioning a rod across the abdominal incision between the loop of the body vessel and the abdomen of the patient to inhibit the loop of the body vessel from receding back into the abdominal cavity of the patient through the abdominal incision;
   detachably supporting the rod by connecting first and second anchoring portions to respective ends of the rod;
   making a first incision into the body vessel to define a first opening within the body vessel;
   making a second incision into the body vessel to define a second opening within the body vessel; and
   replacing the first and second anchoring portions with third and fourth anchoring portions having a profile different than a profile of the first and second anchoring portions.
17. The surgical method according to paragraph 16, wherein replacing the first and second anchoring portions includes providing the third and fourth anchoring portions each having a concavity.
18. The surgical method according to paragraph 16, wherein replacing the first and second anchoring portions includes providing the third and fourth anchoring portions each having a convexity.
19. The surgical method according to paragraph 16, further comprising securing the first and second anchoring portions to the abdomen of the patient by an adhesive on padding portions on the first and second anchoring portions.
20. The surgical method according to paragraph 16, further comprising suturing a portion of the body vessel to the abdomen of the patient.

## Claims

1. A stomal device comprising:
a rod configured to be positioned in a loop of a body vessel to support at least a portion of the body vessel on an abdominal wall, the rod including first and second end portions having respective first and second connecting portions; and
first and second anchoring portions configured to detachably support the rod, the first and second anchoring portions including third and fourth connecting portions configured to detachably mate with the first and second connecting portions of the first and second end portions of the rod, respectively, the first and second anchoring portions transversely extending from the rod.

2. The stomal device according to claim 1, wherein the first and second anchoring portions define respective longitudinal axes.

3. The stomal device according to claim 1 or claim 2, wherein at least one of the first or second anchoring portions includes an arcuate profile.

4. The stomal device according to claim 3, wherein the arcuate profile of the at least one of the first or second anchoring portions has a concavity.

5. The stomal device according to claim 3, wherein the arcuate profile of the at least one of the first or second anchoring portions has a convexity.

6. The stomal device according to any preceding claim, wherein at least one of the first or second anchoring portions includes a padding portion.

7. The stomal device according to claim 6, wherein the padding portion is formed of a foam.

8. The stomal device according to claim 6, wherein the padding portion includes an adhesive layer.

9. The stomal device according to any preceding claim, wherein the first and second connecting portions of the rod and the third and fourth connecting portions of the first and second anchoring portions include dove-tail configuration.

10. A surgical kit comprising:
a rod configured to be positioned in a loop of a body vessel to support at least the loop of the body vessel on an abdominal wall, the rod including first and second end portions;
a pair of first anchoring portions configured to detachably support the rod, the first anchoring portions transversely connectable with the respective first and second end portions of the rod, the first anchoring portions each defining a longitudinal axis;
a pair of second anchoring portions configured to detachably support the rod, the second anchoring portions detachably connectable with the respective first and second end portions of the rod, the second anchoring portions including an arcuate profile having concavity; and
a pair of third anchoring portions configured to detachably support the rod, the third anchoring portions extending transversely from the respective first and second ends of the rod, the third anchoring portions including an arcuate profile having convexity.

11. The surgical kit according to claim 10, wherein the first anchoring portions include connecting portions configured to detachably mate with the respective first and second end portions of the rod.

12. The surgical kit according to claim 10 or claim 11, wherein the second or third anchoring portions include connecting portions configured to detachably mate with the respective first and second end portions of the rod.

13. The surgical kit according to claim 12, wherein each of the first anchoring portions includes padding portions at opposing ends thereof.

14. The surgical kit according to claim 13, wherein the padding portions are formed of a foam.

15. The surgical kit according to claim 13 or claim 14, wherein each of the padding portions includes an adhesive layer.
